# EUROPÄISCHE PATENTANMELDUNG

(11) **EP 0 764 622 A1**
(43) Veröffentlichungstag der Anmeldung: **26.03.1997**
(21) Anmeldenummer: 96114064.7
(22) Anmeldetag: 03.09.1996
(51) Int. Cl.: C07B 31/00

(54) **Verfahren zur Hydrogenolyse von C-O und C=O Bindungen in organischen Substanzen**

(30) Priorität: 23.09.1995 DE 19535395
(71) Anmelder: Degussa Aktiengesellschaft, 60311 Frankfurt (DE)
(72) Erfinder: Lansink-Rotgerink, Hans, Dr., 63864 Glattbach (DE); Scholz, Mario, Dr., 63584 Gründau (DE); Freund, Andreas, Dr., 63801 Kleinostheim (DE); Kunz, Günther, 63486 Bruchköbel (DE)

(57) **Zusammenfassung**

Die Erfindung betrifft ein Verfahren zur Hydrogenolyse von C-O und C=O Bindungen in organischen Substanzen durch Reaktion mit Wasserstoff in Gegenwart eines Katalysatorsystems, welches mindestens ein Metall der Gruppe VIII und/oder IB des periodischen Systems der Elemente auf einem Träger aus mindestens einer Feststoffsäure mit einem Säureaktivitätswert von wenigstens 90 % enthält.

## Beschreibung

Die Erfindung betrifft ein Verfahren zur Hydrogenolyse von C-O und C=O Bindungen in organischen Substanzen durch Reaktion mit Wasserstoff in Gegenwart eines Katalysatorsystems. Bevorzugt handelt es sich bei den organischen Substanzen um Verbindungen von Typ I oder Typ II mit den folgenden Strukturformeln:

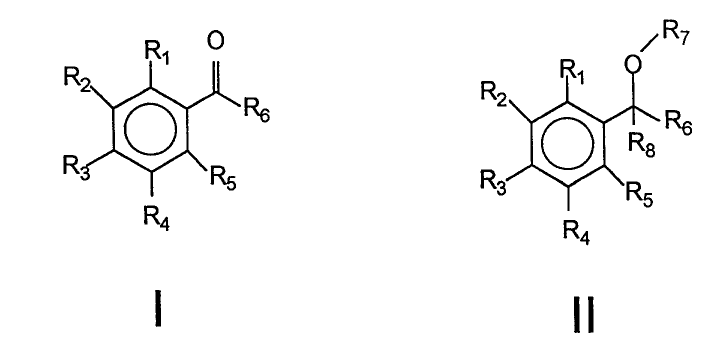

Durch Hydrogenolyse der C=O bzw. C-O Bindungen entstehen daraus die Verbindungen vom Typ III bzw. IV:

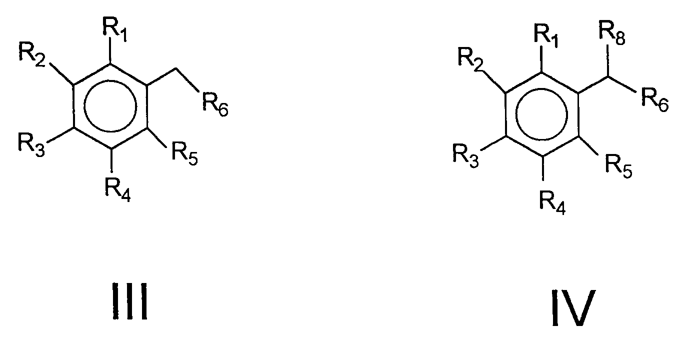

Zur Durchführung dieser Reaktionen werden bislang die Verbindungen vom Typ I oder II in Gegenwart eines festen Hydrierkatalysators in einem Gemisch aus einem Lösungsmittel und einer flüssigen Säure mit molekularem Wasserstoff oder mit einem Wasserstoffdonor in Kontakt gebracht.

Wie im US-Patent 5,124,489 beschrieben, enthält der Hydrierkatalysator im allgemeinen ein Metall der Gruppe VIII, wie z.B. Pd, Pt oder Ni. Dieses Metall ist häufig auf einem Katalysatorträger aufgebracht. Als geeignete Katalysatorträger sind Aluminiumoxid, Siliciumdioxid und Aktivkohle bekannt. Insbesondere die Kombination von Pd auf Aktivkohle (Pd/C) wird für diese Reaktionen häufig angewendet.

Weitere Beispiele für derartige Reaktionen sind im US Patent Nr. 5,047,592 aufgeführt (Beispiele 1 und 2). In diesen Beispielen wird als Hydrierkatalysator 10 Gew-% Pd/C eingesetzt und als Säure verdünnte Salzsäure genommen. Andere Beispiele, in denen Acetale in Gegenwart von Salzsäure und einem 5 oder 10 Gew-% Pd/C Katalysator umgesetzt werden, sind im US Patent Nr. 5,124,489 beschrieben (Beispiele 2,3,4,5,7 und 9).

Die flüssige Säure kann eine Mineralsäure, eine organische Säure oder eine Lewis-Säure sein. Beispiele sind Schwefelsäure, Salzsäure, Phosphorsäure, Essigsäure, Ameisensäure, Benzolsulfonsäure, Toluolsulfonsäure, Methansulfonsäure, Aluminiumtrichlorid usw. Wegen dieser Säuren ist es notwendig, in einer teuren, korrosionsbeständigen Anlage zu arbeiten. Außerdem fällt nach Beendigung der Reaktion ein säurehaltiger Abfallstrom an, der entsorgt werden muß.

Durch den Einsatz der obengenannten Säuren fallen die Reaktionsprodukte häufig als Salze an.

Aufgabe der vorliegenden Erfindung ist es, ein Verfahren zur Hydrogenolyse von C-O oder C=O Bindungen in organischen Substanzen anzugeben, welches ohne korrosive Medien arbeitet und somit auch die Entsorgung von Abfallströmen weitgehend vereinfacht. Darüber hinaus sollen die katalytische Aktivität und Selektivität gegenüber den aus dem Stand der Technik bekannten Verfahren verbessert werden.

Diese Aufgabe wird durch ein Verfahren zur Hydrogenolyse von Verbindungen von Typ I oder II zu Verbindungen vom Typ III oder IV durch Reaktion mit Wasserstoff in Gegenwart eines Katalysatorsystems gelöst, welches dadurch gekennzeichnet ist, daß als Katalysatorsystem ein Feststoffkatalysator eingesetzt wird, welcher mindestens ein Metall der Gruppe VIII und/oder IB des periodischen Systems der Elemente auf einem Träger aus mindestens einer Feststoffsäure mit einem Säureaktivitätswert von wenigstens 90 % enthält.

Die vorliegende Erfindung hat gegenüber dem geschilderten Stand der Technik wesentliche Vorteile. Das Verfahren kann ohne Zusatz von flüssigen Säuren sehr effektiv durchgeführt werden. Dadurch werden die Korrosionsprobleme in der Anlage minimiert. Die Entsorgung von Säure entfällt. Außerdem fällt das Reaktionsprodukt nicht als Salz an. Darüber hinaus ist das erfindungsgemäße Katalysatorsystem mehrfach recyclierbar, d.h. es kann mehrfach eingesetzt werden.

In den Verbindungen vom Typ I, II, III und IV können R₁ bis R₈, unabhängig voneinander, Wasserstoff (H), Alkylgruppen, substituierte oder unsubstituierte Arylgruppen, Hydroxygruppen, Alkoxygruppen, substituierte oder unsubstituierte Aryloxygruppen, Halogengruppen, Carbonsäuregruppen, Carbonsäurederivatgruppen, Acyloxygruppen, Nitrogruppen, Sulfonsäuregruppen, Mercaptogruppen, Aminogruppen, alkylsubstituierte Aminogruppen und substituierte oder unsubstituierte arylsubstituierte Aminogruppen sein. Im Falle von Alkylgruppen können diese 1 bis 20 C-Atome umfassen, bevorzugt 1-6 C-Atome. Im Falle der substituierten oder unsubstituierten Arylgruppen bestehen diese bevorzugt aus Phenylgruppen.

Gemäß der hier beschriebenen Erfindung werden die Verbindungen vom Typ I oder II entweder unverdünnt oder gelöst in einem Lösungsmittel ohne zusätzliche flüssige Säure im Reaktor mit dem festen Katalysator in Kontakt gebracht. Wasserstoff wird entweder als molekularer Wasserstoff oder in Form eines Wasserstoffdonors zugeleitet oder hinzugefügt.

Der feste Katalysator enthält mindestens ein Hydriermetall sowie mindestens eine saure Komponente. Die saure Komponente dient gleichzeitig als Feststoffsäure und als Träger für das Hydriermetall. Als Hydriermetall kommen die Metalle der Gruppen VIII (Fe, Co, Ni, Ru, Rh, Pd, Os, Ir und Pt) und IB (Cu, Ag und Au) in Betracht. Bevorzugt werden jedoch Rh, Pd, Pt oder Ni eingesetzt. Insbesondere mit Pd wurden gute Ergebnisse erzielt. Die katalytische Wirkung des Hydriermetalls oder der Hydriermetalle kann durch Zusatz von Promotoren modifiziert werden. Als Promotoren können z.B. die Elemente der Gruppe IVA (Sn, Ge, Pb) oder Indium eingesetzt werden.

Der Metallgehalt kann zwischen 0.01 und 90 Gew-%, bezogen auf das Gesamtgewicht des Katalysators, liegen. Bei Verwendung von Edelmetall-Katalysatoren liegt der bevorzugte Metallgehalt im Bereich zwischen 0,01 und 30 Gew.-%. Im Falle der Nichtedelmetall-Katalysatoren kann der Metallgehalt deutlich höher liegen. Bei Verwendung eines cogefällten Nickel-Aluminiumoxid-Katalysators werden zum Beispiel bei Metallgehalten von bis zu 90 Gew.-% noch gute Metalldispersionen erhalten (H.G.J. Lansink-Rotgerink et al. in Applied Catalysis, Vol. 27, (1986), Seite 41). Der Katalysator kann entweder in seiner reduzierten Form vorgelegt werden oder in-situ während der Durchführung des erfindungsgemäßen Verfahrens reduziert werden.

Die als Träger für die Hydriermetalle dienende saure Komponente des Katalysators ist eine Feststoffsäure mit einem Säureaktivitätswert von mindestens 90%. Der Säureaktivitätswert wird in einem Test ermittelt, bei dem ein Gasstrom, der aus 5 Vol.-% Isopropanol und 95 Vol.-% Helium besteht, durch ein Katalysatorbett geleitet wird. Die Katalysatormenge im Reaktor beträgt 1.00 g, der Katalysator besteht aus Partikel im Bereich zwischen 0.5 - 2.0 mm. Der Helium-Durchsatz durch den Reaktor beträgt 50 ml/min. Das Isopropanol wird durch die Feststoffsäure zersetzt. Als primäre Hauptprodukte entstehen dabei Propen und Wasser. Je nach Feststoffsäure können Folgeprodukte gebildet werden. Der Säureaktivitätswert im Rahmen dieser Erfindung ist als der Umsetzungsgrad von Isopropanol bei einer Temperatur von 250°C definiert.

Für die Durchführung des erfindungsgemäßen Verfahrens ist ein Säureaktivitätswert des Katalysatorträgers nahe 100 % wünschenswert. Mit sinkendem Säureaktivitätswert nimmt die Aktivität des Katalysatorsystems für die Hydrogenolyse der C-O und C=O Bindungen rasch ab. Unterhalb eines Säureaktivitätswertes von 90 % ist das Verfahren nicht mehr wirtschaftlich sinnvoll durchführbar.

Beispiele von Feststoffsäuren sind: amorphe, Silicium und Aluminium enthaltende Mischoxide, kristalline Verbindungen auf der Basis von Si, Al und O. Die Verbindungen können noch in bekannter Weise durch Tränken mit Säuren modifiziert werden. Zur Modifizierung eignen sich zum Beispiel Schwefel- oder Phosphorsäure.

Bei den genannten Verbindungen handelt es sich lediglich um eine kleine Auswahl geeigneter Feststoffsäuren. Für einen Überblick über das Gebiet der Feststoffsäuren wird auf das Buch "Studies in Surface Science and Catalysis, Vol. 51, (New Solid Acids and Bases)" von K. Tanabe et al. (Elsevier Science Publishers, Amsterdam, 1989) verwiesen.

Das SiO₂/Al₂O₃ Molverhältnis der amorphen Si-Al-Mischoxide kann von 99/1 bis 1/99, bevorzugt von 95/5 bis 5/95 variieren.

Die kristallinen Verbindungen auf Basis Si, Al und O umfassen die Gruppe der Zeolithe, wie z.B. ZSM-5, Mordenit, Zeolith-Beta oder Zeolith-Y. Das SiO₂/Al₂O₃-Molverhältnis der Zeolithen kann von 2 bis unendlich variieren. Die Zeolithe werden bevorzugt in der H-Form eingesetzt.

Das erfindungsgemäße Verfahren wird bei Temperaturen zwischen -20 und 500°C durchgeführt. Wenn molekularer Wasserstoff verwendet wird, kann der Wasserstoffdruck zwischen 0.1 und 100 bar variieren, je nach Art des Edukts. Wasserstoff kann auch durch einem Wasserstoffdonor in-situ geliefert werden.

Die Reaktion kann sowohl in einem Batchprozeß, in einem semikontinuierlichen Prozeß als auch in einem kontinuierlichen Prozeß durchgeführt werden. Das Verfahren kann sowohl in Suspension als auch im Festbett durchgeführt werden. Im ersten Fall liegt der Katalysator als Pulver vor, in dem letzten Fall als Festkörper mit Abmessungen von mindestens 0.3 mm. Im Falle eines Festbettverfahrens kann die Reaktion sowohl in der Flüssigphase, in der Gasphase als auch in einer überkritischen Phase stattfinden.

Das bei der Reaktion entstehende Wasser kann gegebenenfalls durch Adsorption oder durch einen Wasserabscheider, z.B. nach Dean, abgetrennt werden.

### Beispiel 1

Zur Durchführung des erfindungsgemäßen Verfahrens wurden Palladium-Katalysatoren mit jeweils 2 Gew.-% Palladium auf verschiedenen Trägermaterialien hergestellt. Hierzu wurden die Trägermaterialien mit einer Lösung aus Palladiumchlorid imprägniert, abfiltriert, chlorfrei gewaschen, getrocknet, bei 400°C unter einer Stickstoffatmosphäre calciniert und abschließend in einem Strom aus Formiergas (5 Vol.-% H₂, 95 Vol.-% N₂) bei 400°C reduziert. Die reduzierten Katalysatoren wurden trocken an Luft aufbewahrt.

Die so hergestellten Katalysatoren A bis D sind in der Tabelle 1 durch Angabe der Trägermaterialien, ihrer Restgehalte an Na₂O, ihrer SiO₂/Al₂O₃-Molverhältnisse und der gemessenen Säureaktivitätswerte charakterisiert.

In Tabelle 1 ist mit Katalysator E auch ein kommerzieller Palladium-Katalysator auf Aktivkohle aufgenommen worden, der in den folgenden Verfahrensbeispielen zu Vergleichszwecken mit den Verfahren nach dem Stand der Technik herangezogen wurde.

**Tabelle 1**

| Palladium-Katalysatoren (2 Gew.-% Pd) auf verschiedenen Trägermaterialien | | | | |
|---|---|---|---|---|
| Katalysator | Träger | | | |
| | Material | Na₂O [Gew.-%] | SiO₂/Al₂O₃ [mol/mol] | Säureaktivität [%] |
| A | ZSM-5; H-Form | 0,01 | 27,4 | 100 |
| B | Mordenit H-Form | 0,4 | 29,6 | 100 |
| C | Zeolith-Beta H-Form | 0,05 | 27 | 100 |
| D | Zeolith Y H-Form | 0,1 | 27,4 | 100 |
| E | 5 Gew.-% Pd/C; kommerzieller Katalysator für Vergleichsversuche | | | |

### Beispiel 2

Das erfindungsgemäße Verfahren wurde beispielhaft bei der Umsetzung von 1-Phenylethanol mit Wasserstoff zu Ethylbenzol unter Verwendung der Katalysatoren A bis D getestet. Hierzu wurden jeweils 5 g 1-Phenylethanol (entsprechend 40,93 mmol) mit 115 ml Ethanol verdünnt und unter Zugabe des Katalysators in einen mit Wasserstoffgas gefüllten Reaktor eingefüllt. Die Katalysatormenge wurde so bemessen, daß im Reaktor jeweils 100 mg Palladium für die Hydrierung zur Verfügung standen.

Katalysator E wurde zur Durchführung der Umsetzung von 1-Phenylethanol mit Wasserstoff zu Ethylbenzol gemäß den aus dem Stand der Technik bekannten Verfahren eingesetzt. Die Katalysatormenge wurde ebenfalls so bemessen, daß im Reaktor 100 mg Palladium für die Hydrierung zur Verfügung standen. Dem Reaktionsgemisch wurden in diesem Fall 12 ml einer 46 gewichtsprozentigen Schwefelsäure als flüssige Säure zugefügt. Ohne Säurezusatz fand mit Katalysator E keine nennenswerte Umsetzung des 1-Phenylethanols statt.

Die Umsetzung wurde bei 30°C und einem Wasserstoffdruck von 1,01 bar (abs.) unter ständigem Rühren durchgeführt. Nach jeweils 5 Stunden wurde die Reaktion abgebrochen, der Katalysator vom Reaktionsgemisch getrennt und das Reaktionsgemisch mittels Gaschromatographie unter Verwendung eines FID-Detektors analysiert. Die Ergebnisse sind in Tabelle 2 zusammengefaßt. Bei den Prozentzahlen in Tabelle 2 handelt es sich um die Flächenprozente der betreffenden Substanz nach Abzug des Lösungsmittelpeaks.

Wie aus der Tabelle 2 hervorgeht, sind die Katalysatoren A bis D auch ohne Säurezusatz deutlich aktiver bzw. selektiver bei der Umsetzung von 1-Phenylethanol zu Ethylbenzol als der Katalysator E mit Säurezusatz.

**Tabelle 2**

| Hydrogenolyse von 1-Phenylethanol mit den Katalysatoren A bis E | | | | | | |
|---|---|---|---|---|---|---|
| Katalysator | Typ | Säurezusatz | 1-Phenylethanol [Flächen-%] | Ethylbenzol [Flächen-%] | Acetophenon [Flächen-%] | Ethylether des 1-Phenylethanols [Flächen-%] |
| A | 2% Pd, ZSM-5 | nein | 29,7 | 69,9 | 0,4 | 0,0 |
| B | 2% Pd, MOR | nein | 12,8 | 87,0 | 0,2 | 0,0 |
| C | 2% Pd, BETA | nein | 6,5 | 93,0 | 0,3 | 0,2 |
| D | 2%Pd, Y | nein | 4,7 | 94,4 | 0,6 | 0,3 |
| E | 5% Pd, C | ja | 3,0 | 73,2 | 23,4 | 0,4 |
| MOR: Mordenit; BETA: Zeolith Beta; Y: Zeolith Y; | | | | | | |

### Beispiel 3

Analog zu Beispiel 1 wurden zwei weitere Palladium-Katalysatoren auf Zeolith-Beta (H-Form, Na₂O-Gehalt: 0.05 Gew-%, SiO₂/Al₂O₃ = 27 mol/mol, Säureaktivitätswert 100%) hergestellt. Die Menge des aufgebrachten Palladiums wurde variiert. Alle anderen Parameter waren identisch zu denen in Beispiel 1.

Der so enthaltene Katalysator F enthielt 5 Gew-% Palladium und Katalysator G enthielt 20 Gew-% Palladium.

### Beispiel 4

Die Katalysatoren C, F und G wurden wie in Beispiel 2 zur Hydrogenolyse von 1-Phenylethanol eingesetzt. Die eingesetzte Menge an Pd betrug wie im Beispiel 2 jeweils 100 mg, d.h. die Katalysatormenge der Katalysatoren F und G wurde entsprechend ihrer Palladium-Beladung verringert. Die Ergebnisse sind in Tabelle 3 zusammengefaßt.

**Tabelle 3**

| Hydrogenolyse von 1-Phenylethanol mit den Katalysatoren C, F und G | | | | | |
|---|---|---|---|---|---|
| Katalysator | Pd [Gew.-%] | 1-Phenylethanol [Flächen-%] | Ethylbenzol [Flächen-%] | Acetophenon [Flächen-%] | Ethylether des 1-Phenylethanols [Flächen-%] |
| C | 2 | 6,5 | 93,0 | 0,3 | 0,2 |
| F | 5 | 8,4 | 88,8 | 2,8 | 0,0 |
| G | 20 | 11,7 | 86,4 | 1,7 | 0,2 |

Wie aus Tabelle 3 hervorgeht, nimmt sowohl die Aktivität als auch die Selektivität zum gewünschten Produkt mit zunehmenden Palladium-Gehalt leicht ab. Aktivität und Selektivität bleiben aber deutlich höher als beim konventionellen Verfahren mit Katalysator E.

### Beispiel 5

Es wurde versucht, 1-Phenylethanol ohne Zusatz von Wasserstoff mit Katalysator F umzusetzen. Der Test wurde analog zum Beispiel 2 durchgeführt, jedoch wurde an Stelle von Wasserstoff mit Stickstoff gespült. Das Ergebnis ist in Tabelle 4 festgehalten.

**Tabelle 4**

| Testergebnisse für die Hydrogenolyse von 1-Phenylethanol mit Katalysator F Einfluß von Wasserstoff | | | | | |
|---|---|---|---|---|---|
| Katalysator | Gasphase | 1-Phenylethanol [Flächen-%] | Ethylbenzol [Flächen-%] | Acetophenon [Flächen-%] | Ethylether des 1-Phenylethanols [Flächen-%] |
| F | N₂ | 80,9 | 8,7 | 10,3 | 0,1 |
| F | H₂ | 8,4 | 88,8 | 2,8 | 0,0 |

Aus Tabelle 4 geht hervor, daß auch ohne Zusatz von Wasserstoff ein Teil des Edukts in das gewünschte Produkt umgesetzt wird. Es findet hierbei ein Wasserstoff-Transfer von 1-Phenylethanol (H-Donor) zu Ethylbenzol (H-Akzeptor) unter gleichzeitiger Bildung von Acetophenon statt. Der Umsatz und die Selektivität sind jedoch viel geringer als bei Anwesenheit von gasförmigen Wasserstoff.

### Beispiel 6

Analog zu Beispiel 1 wurde ein weiterer Palladiumkatalystor (Katalysator H) auf pyrogener Kieselsäure (Degussa Aerosil-200; > 99 Gew.-% SiO₂) hergestellt. Im Unterschied zu den Trägern von Beispiel 1 weist pyrogene Kieselsäure nur einen Säureaktivitätswert von etwa 30 % auf.

Katalysator H wurde analog zu Beispiel 2 getestet. Das Ergebnis ist in der untenstehende Tabelle festgehalten.

**Tabelle 5**

| Testergebnisse für die Hydrogenolyse von 1-Phenylethanol mit Katalysator H | | | | | | |
|---|---|---|---|---|---|---|
| Katalysator | Typ | Säurezusatz | 1-Phenylethanol [Flächen-%] | Ethylbenzol [Flächen-%] | Acetophenon [Flächen-%] | Ethylether des 1-Phenylethanols [Flächen-%] |
| H | 2% Pd, SiO₂ | nein | 89,8 | 9,0 | 0,0 | 0,0 |

Wie aus dem Ergebnis zu sehen ist, hat Katalysator H mit einem Säureaktivitätswert von 30% eine viel geringere Aktivität als die anderen Katalysatoren, die unter den gleichen Bedingungen eingesetzt wurden.

## Patentansprüche

1. Verfahren zur Hydrogenolyse von Verbindungen vom Typ I oder II zu Verbindungen vom Typ III oder IV 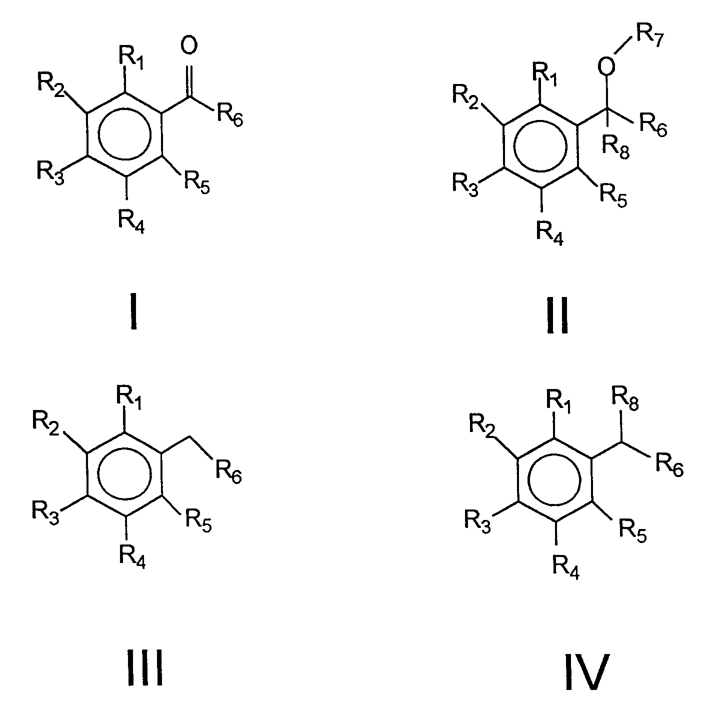 durch Reaktion mit Wasserstoff in Gegenwart eines Katalysatorsystems,
**dadurch gekennzeichnet**,
daß als Katalysatorsystem ein Feststoffkatalysator eingesetzt wird, welcher mindestens ein Metall der Gruppe VIII und/oder IB des periodischen Systems der Elemente auf einem Träger aus mindestens einer Feststoffsäure mit einem Säureaktivitätswert von wenigstens 90% enthält.

2. Verfahren nach Anspruch 1,
**dadurch gekennzeichnet**,
daß der Katalysator mindestes eines der Metalle Palladium, Platin, Rhodium und Nickel enthält.

3. Verfahren nach den Ansprüchen 1 bis 2,
**dadurch gekennzeichnet**,
daß die Feststoffsäure zu der Gruppe der Zeolithe gehört.

4. Verfahren nach den Ansprüchen 1 bis 3,
**dadurch gekennzeichnet**,
daß als Zeolith ZSM-5, Y, Beta oder Mordenit verwendet wird.

5. Verfahren nach den Ansprüchen 1 bis 2,
**dadurch gekennzeichnet**,
daß als Feststoffsäure ein nicht zeolithisches Material verwendet wird.

6. Verfahren nach den Ansprüchen 1, 2 und 5,
**dadurch gekennzeichnet**,
daß als nicht zeolithisches Material mindestens ein Mischoxid verwendet wird.

7. Verfahren nach den Ansprüchen 1, 2, 5 und 6,
**dadurch gekennzeichnet**,
daß das Mischoxid mit einer Säure modifiziert ist.

8. Verfahren nach den Ansprüchen 1 bis 7,
**dadurch gekennzeichnet**,
daß R₁ bis R₈, unabhängig voneinander, Wasserstoff (H), Alkylgruppen, substituierte oder unsubstituierte Arylgruppen, substituierte oder unsubstituierte Phenylgruppen, Hydroxygruppen, Alkoxygruppen, substituierte oder unsubstituierte Aryloxygruppen, Halogengruppen, Carbonsäuregruppen, Carbonsäurederivatgruppen, Acyloxygruppen, Nitrogruppen, Sulfonsäuregruppen, Mercaptogruppen, Aminogruppen, alkylsubstituierte Aminogruppen oder substituierte oder unsubstituierte arylsubstituierte Aminogruppen sind.
